(19) Europäisches Patentamt

European Patent Office

Office européen des brevets

(11) Numéro de publication : **0 323 321 B1**

(12) **FASCICULE DE BREVET EUROPEEN**

(45) Date de publication du fascicule du brevet :
**18.03.92 Bulletin 92/12**

(21) Numéro de dépôt : **88403278.0**

(22) Date de dépôt : **22.12.88**

(51) Int. Cl.⁵ : **A61M 5/14**

(54) Perfectionnement aux pousse-seringues.

(30) Priorité : **24.12.87 FR 8718466**

(43) Date de publication de la demande :
**05.07.89 Bulletin 89/27**

(45) Mention de la délivrance du brevet :
**18.03.92 Bulletin 92/12**

(84) Etats contractants désignés :
**DE GB IT NL**

(56) Documents cités :
**EP-A- 0 204 977**
**EP-A- 0 229 450**
**DE-C- 3 340 511**
**FR-A- 2 546 068**
**US-A- 3 964 139**

(73) Titulaire : **Godefroy, Alain Jean Robert**
**19, chemin des picardes**
**F-78510 Triel (FR)**

(72) Inventeur : **Godefroy, Alain Jean Robert**
**19, chemin des picardes**
**F-78510 Triel (FR)**

(74) Mandataire : **Lerner, François**
**LERNER & BRULLE S.C.P. 5, rue Jules**
**Lefèbvre**
**F-75009 Paris (FR)**

EP 0 323 321 B1

**Description**

La présente invention concerne un appareil du type pousse-seringue.

Les appareils désignés par le vocable "pousse-seringues" permettent d'effectuer des injections de produits liquides contenus dans une seringue manuelle classique, sans autre intervention que leur mise en oeuvre au départ de l'injection. Qu'ils soient monoseringue ou qu'ils permettent l'utilisation simultanée de plusieurs seringues, les appareils pousse-seringues existants sont spécifiques d'un type prédéterminé de seringue ou, au mieux, d'un nombre limité de types déterminés de seringues du commerce. Ils présentent en outre l'inconvénient de ne pas assurer un centrage correct du piston sur l'axe du corps de la seringue, ce qui provoque un arrêt intempestif de l'avance de ce piston, par coincement de ce dernier dans le corps de la seringue.

La pression d'injection ne doit pas dépasser une limite au-delà de laquelle il faut arrêter l'injection et alerter l'utilisateur ; or cette pression est fonction du carré de diamètre intérieur de la seringue, et la plupart des appareils actuels ne tiennent pas compte de l'importance de variation de pression d'occlusion suivant la seringue utilisée. Certains appareils prévoient un réglage manuel en fonction du type de seringue, mais cela constitue une servitude supplémentaire à l'emploi.

Les pousse-seringues prévus pour recevoir plus d'une seringue peuvent n'en recevoir qu'une, mais ils sont encombrants et plus onéreux que les appareils monoseringue.

Selon le brevet EP-A-0 204 977 on connaît déjà un appareil comprenant plusieurs pousse-seringues qui peuvent être montés côte à côte dans un boîtier de façon à être simultanément actionnés par un moteur de commande contrôlé par un dispositif électronique convenablement programmé. Mais dans cet appareil, les pousse-seringues ne sont pas mécaniquement indépendants les uns des autres et il n'y a pas de dissociation entre la fonction de commande électronique et la fonction de commande mécanique des pousse-seringues individuels.

Le pousse-seringue, objet de la présente invention, présente des caractéristiques telles que les inconvénients précités sont éliminés.

L'appareil du type pousse-seringue conforme à l'invention se caractérise en ce qu'il est constitué par un élément indépendant comprenant un dispositif de fixation de seringue et l'ensemble mécanique complet d'entraînement du piston de cette seringue, juxtaposé à un élément de commande et de contrôle électronique de l'entraînement du piston, ces éléments étant maintenus entre-eux par un mécanisme de fixation amovible assurant la liaison mécanique et électronique entre ces éléments. De cette manière est constitué un pousse-seringue dans lequel la commande de chaque pousse-seringue peut être commandée indépendamment des autres et cela à partir d'un seul boîtier électronique de commande lié mécaniquement et électroniquement à tous les pousse-seringues de l'appareil.

Des modes avantageux de réalisation de l'invention sont décrits dans les revendications dépendantes.

L'invention est décrite ci-après plus en détail à l'aide de dessins qui ne représentent que des modes d'exécution non limitatifs de l'invention.

La figure 1 représente en perspective un schéma de principe de l'invention.

La figure 2 est une vue schématique du dispositif de centrage de la seringue selon l'invention.

La figure 3 est une vue schématique en coupe de la figure 2.

Les figures 4 et 5 sont des vues schématiques du dispositif de centrage de la tête de piston.

Les figures 6 et 7 sont des vues schématiques d'un détecteur de pression d'occlusion.

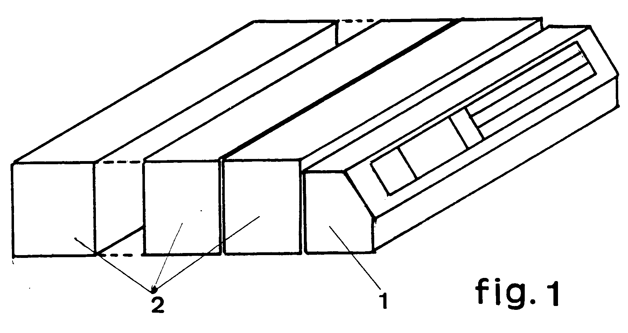

La figure 1 schématise le principe de cette disposition : le block 1 représente la partie commune à toutes les combinaisons et son rôle est d'assurer la "gestion" de l'appareil. Les blocs 2 représentent chacun un pousse-seringue monoseringue autonome, à l'exception de l'asservissement de vitesse d'injection, lequel est fourni par le bloc commun de commande et de contrôle 1, à partir des instructions qui ont été affichées. Chaque bloc 2 comprend aussi un dispositif d'étalonnage du débit, en fonction de la seringue mise en place, et un système de fixation du corps et du piston de la seringue, assurant un centrage correct de ces deux éléments, pendant toute la durée de l'injection, et cela quel que soit le type de seringue utilisé. Le nombre maximum de blocs 2 ne dépend que de la capacité de traitement du système de commande 1.

Les blocs 2 sont réunis entre eux, et le premier au bloc 1 par un dispostif classique de verrouillage et les connexions électriques assurées par des connecteurs fixes solidaires des blocs ; l'interchangeabilité est ainsi assurée sans intervention importante et peut être faite directement par l'utilisateur lui-même.

Le bloc de commande et de contrôle comporte en face avant tous les éléments permettant de programmer la marche individuelle de chacun des blocs 2, et de suivre le bon fonctionnement de l'ensemble.

Dans des variantes plus simples, les ordres peuvent être exécutés directement, mais le principe de la disposition modulaire en éléments assemblés et amovibles de la figure 1 est conservé.

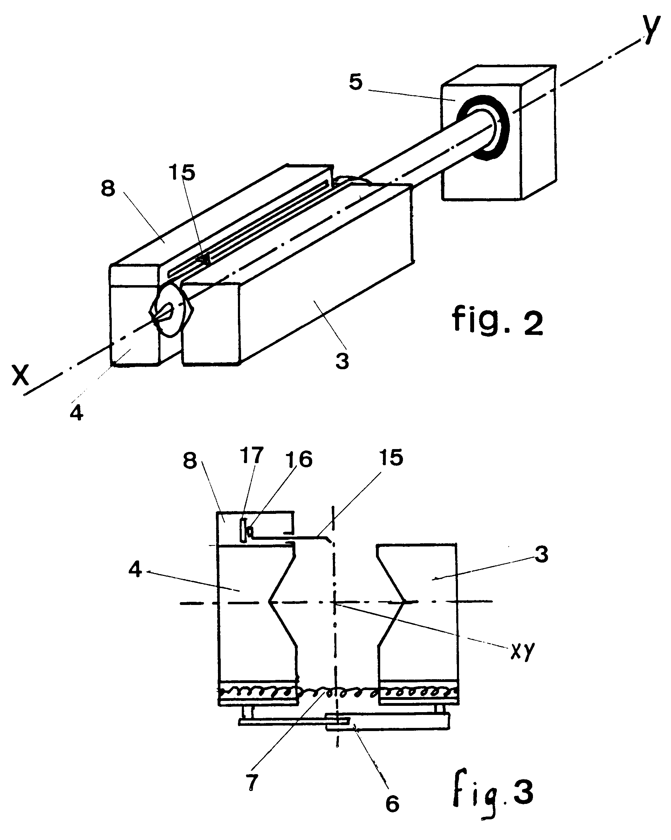

Le dispositif de centrage automatique du corps de la seringue et de son piston comporte deux éléments constitués schématisés figure 2, dans laquelle des mâchoires 3 et 4 maintiennent toujours le corps de la seringue sur son axe de référence XY et ce, quel que soit le diamètre de cette seringue ; la fixation de la seringue est complétée par un poussoir 5 décrit plus loin, associé ou non. Les mâchoires 3 et 4, liées entre-elles par un mécanisme approprié, par exemple de jeu de biellettes ou d'engrenages 6, sont liées par un ressort 7 qui tend à les approcher, et permet le maintien de la seringue en place.

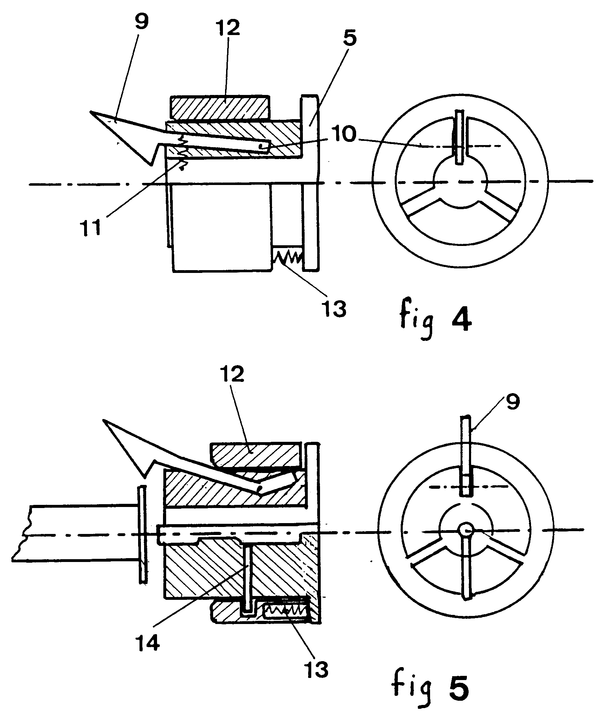

Sur une des deux mâchoires 3 ou 4, est fixé le dispositif d'étalonnage qui permet de déterminer la vitesse d'avance du piston de la seringue, en fonction des graduations de celle-ci, suivant un procédé connu. La figure 3 représente, en coupe, les mâchoires 3 et 4 ainsi que le dispositif d'étalonnage 8.

Le centrage de la tête du piston de la seringue est assuré par la forme concave du poussoir 5 constitué, dans une réalisation, par un cône dont l'axe de symétrie est confondu avec l'axe de référence XY. Dans la pratique, la tête du piston de la seringue devant être maintenue contre son poussoir afin qu'il ne puisse pas avancer indépendamment de celui-ci, comporte, dans une réalisation préférée (figure 4), un cône constitué par des crochets 9, au nombre minimum de trois, et pouvant se déplacer dans le plan de l'axe XY, autour d'un axe 10 perpendiculaire à ce plan ; un jeu de ressorts 11 les maintient en position fermée. La forme de ces crochets est telle qu'ils viennent automatiquement se mettre en place autour de la tête du piston de la seringue, à l'approche du poussoir, et cela indépendamment du diamètre de cette tête. Pour assurer le centrage de ce système de crochets, une bague circulaire 12, poussée par un ressort 13 vient bloquer les crochets 9 dans leur position de maintien. Dans une des réalisations préférées les crochets 9 comportent une partie postérieure de forme telle que la bague 12 les maintient en position écartée, lorsqu'elle est en position arrière, tel que schématisé figure 5 ; cette disposition permet en outre de libérer aisément la tête du piston de seringue pour dégager celle-ci. Enfin dans cette réalisation préférée, mais non exclusive, la bague 12 est retenue en position arrière par un verrou à échappement 14 qui la libère quand la tête de piston de seringue vient appuyer sur le poussoir 5. Avec cette disposition, les ressorts de rappel 11 ne sont plus utiles, la bague 12 assurant le maintien centré des crochets.

Le dispositif d'étalonnage 8 est connu et constitué par un index 15 déplaçable parallèlement au corps de la seringue, le long de sa graduation, et entraînant un curseur 16 sur un élément rectiligne de repérage de position 17 dont les indications sont transmises au microprocesseur de gestion.

Du fait que le mécanisme de poussée du piston de seringue lui applique une force axiale dont le maximum est sensiblement constant, et que ce maximum correspond à la pression atteinte dans la seringue en cas d'occlusion de la tubulure d'administration du produit injecté, cette pression d'occlusion dépend du carré du diamètre de la seringue ; or il est impératif de maintenir cette pression maximale d'occlusion en-dessous d'une limite déterminée, quelle que soit la seringue utilisée.

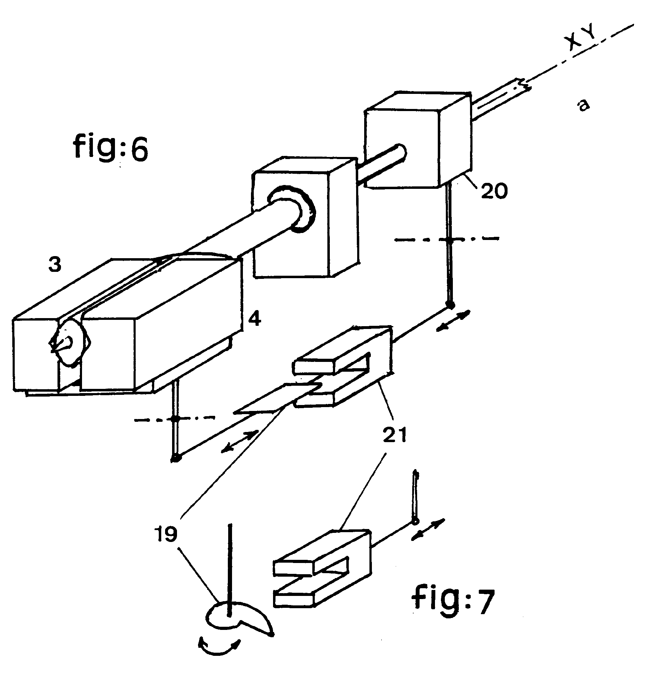

Le dispositif adopté dans l'appareil objet de la présente invention, consiste en un détecteur du rapport de la valeur du diamètre de la seringue à la valeur du couple moteur maximum au moment d'une occlusion. Dans une réalisation préférée, le mouvement relatif des deux mâchoires 3 et 4 de maintien du corps de la seringue entraîne, par un mécanisme (figure 6) un volet obturateur opaque 19 qui vient intercepter le faisceau d'un détecteur photoélectrique de passage 21 et dont la position relative par rapport au volet 19 est commandée par le détecteur de couple moteur 20. Dans la réalisation préférée mais non exclusive (figure 7) le volet obturateur est constitué par un disque tournant autour d'un axe entraîné par le mouvement des mâchoires 3 et 4, et dont le profil extérieur est tel que l'interruption du faisceau du détecteur 21 ait lieu pour une pression d'occlusion donnée, indépendante de la position des mâchoires, donc du diamètre de la seringue utilisée.

## Revendications

1. Appareil du type pousse-seringue constitué par un élément indépendant (2) comprenant un dispositif de fixation de seringue et l'ensemble mécanique complet d'entraînement du piston de cette seringue, juxtaposé à un élément (1) de commande et de contrôle électronique de l'entraînement du piston, ces éléments (1, 2) étant maintenus entre-eux par un mécanisme de fixation amovible assurant la liaison mécanique et électrique entre ces éléments (1, 2).

2. Appareil du type pousse-seringue selon la revendication 1 caractérisé par le fait qu'un ou plusieurs éléments mécaniques (2) peuvent être ajoutés au premier élément mécanique par un système de fixation amovible identique, permettant un contrôle et une commande indépendants pour chacun des éléments mécaniques, et que tous ces éléments sont en conséquence interchangeables.

3. Appareil pousse-seringue selon les revendications 1 et 2, caractérisé par le fait que chaque élément mécanique 2 peut être conçu pour recevoir plusieurs seringues.

## Patentansprüche

1. Spritzenpumpenvorrichtung, bestehend aus einem unabhängigen Element (2), welches eine Spritzenbefestigungsvorrichtung und die gesamte mechanische Einheit für den Antrieb des Kolbens dieser Spritze enthält und mit einem elektronischen Steuer- und Kontrollelement (1) für den Antrieb des Kolbens Seite an Seite verbunden ist, wobei diese Elemente (1, 2) durch eine lösbare Befestigungsvorrichtung, welche die mechanische und elektrische Verbindung zwischen diesen Elementen (1, 2) gewährleistet, in ihrer Stellung zueinander gehalten werden.

2. Spritzenpumpenvorrichtung nach Anspruch 1, dadurch gekennzeichnet, daß ein oder mehrere mechanische Elemente (2) mittels eines identischen lösbaren Befestigungssystems zu dem ersten mechanischen Element hirzugesetzt werden können, so daß die einzelnen mechanischen Elemente unabhängig voneinander kontrolliert und gesteuert werden können und folglich all diese Elemente untereinander austauschbar sind.

3. Spritzenpumpenvorrichtung nach den Ansprüchen 1 und 2, dadurch gekennzeichnet, daß jedes mechanische Element (2) für die Aufnahme mehrerer Spritzen ausgebildet sein kann.

## Claims

1. Apparatus of the syringe-pump type consisting of an independent component (2) comprising a syringe securing device and the complete mechanical assembly for driving the piston of this syringe, adjacent a component (1) for electronically controlling and checking the drive of the piston, these components (1, 2) being held with respect to each other by a detachable securing mechanism which connects these components (1, 2) mechanically and electrically.

2. Apparatus of the syringe-pump type according to Claim 1, characterised in that one or more mechanical components (2) may be added to the first mechanical component by an identical detachable securing system enabling each of the mechanical components to be checked and controlled independently; and in that all these components are therefore interchangeable.

3. Syringe-pump apparatus according to Claims 1 and 2, characterised in that each mechanical component (2) may be designed to received a plurality of syringes.

fig. 1

fig. 2

fig. 3

fig 4

fig 5

fig:6

XY

a

20

3

4

21

19

fig:7